# EUROPÄISCHE PATENTANMELDUNG

(11) **EP 1 304 563 A1**
(43) Veröffentlichungstag der Anmeldung: **23.04.2003**
(21) Anmeldenummer: 02019081.5
(22) Anmeldetag: 28.08.2002
(51) Int. Cl.: G01N 27/327, C12Q 1/00, C12Q 1/68

(54) **Halbleitervorrichtung mit mehrschichtigem Aufbau sowie Verfahren zu dessen Herstellung**

(30) Priorität: 22.10.2001 DE 10152002
(71) Anmelder: Infineon Technologies AG, 81669 München (DE)
(72) Erfinder: Ehben, Thomas, 81379 München (DE); Fritz, Michaela, Dr., 81673 München (DE); Haneder, Thomas, 85221 Dachau (DE)
(74) Vertreter: Müller-Boré & Partner Patentanwälte

(57) **Zusammenfassung**

Die vorliegende Erfindung betrifft eine Halbleitervorrichtung mit mehrschichtigem Aufbau, mindestens umfassend ein Halbleitersubstrat und als äußere Schicht des Mehrschichtaufbaus eine SiO₂-Schicht in einer Dicke von größer als 5 nm, wobei auf der SiO₂-Schicht eine Linkermolekül-Schicht auf der Basis einer Siliciumorganischen Verbindung angeordnet ist und wobei die Siliciumorganische Verbindung kovalent an die SiO₂-Schicht gebunden ist und weiter eine funktionelle Gruppe aufweist, die zur kovalenten Bindung mit als Sonden in biologisch-chemischen Reaktionen einsetzbaren Fängermolekülen befähigt ist, sowie ein Verfahren zu deren Herstellung. Die erfindungsgemäße Halbleitervorrichtung eignet sich als "BioChip-Grundmodul" in Verfahren zum Nachweis biochemischer Reaktionen sowie hierfür insbesondere zur Untersuchung zur DNA-Hybridisierung, Protein-Protein-Wechselwirkungen und anderer Bindungsreaktionen im Bereich der Genom-, Proteom- oder Wirkstoffforschung in Biologie und Medizin.

## Beschreibung

Die vorliegende Erfindung betrifft eine Halbleitervorrichtung mit mehrschichtigem Aufbau, mindestens umfassend ein Halbleitersubstrat und als äußere Schicht des Mehrschichtaufbaus eine SiO₂-Schicht in einer Dicke von größer als 5 nm, wobei auf der SiO₂-Schicht eine Linkermolekül-Schicht auf der Basis einer Silicium-organischen Verbindung angeordnet ist und wobei die Silicium-organische Verbindung kovalent an die SiO₂-Schicht gebunden ist und weiter eine funktionelle Gruppe aufweist, die zur kovalenten Bindung mit als Sonden in biologisch-chemischen Reaktionen einsetzbaren Fängermolekülen befähigt ist, sowie ein Verfahren zu deren Herstellung. Die erfindungsgemäße Halbleitervorrichtung eignet sich als "BioChip-Grundmodul" in Verfahren zum Nachweis biochemischer Reaktionen sowie hierfür insbesondere zur Untersuchung zur DNA-Hybridisierung, Protein-Protein-Wechselwirkungen und anderer Bindungsreaktionen im Bereich der Genom-, Proteom- oder Wirkstoffforschung in Biologie und Medizin.

In der Molekularbiologie finden heute in zunehmendem Maße Biochips Verwendung, mit denen auf schnelle Art und Weise Erkenntnisse über Organismen und Gewebe gewonnen werden.Für die Biowissenschaften und die medizinische Diagnostik ist die Detektion (bio)chemischer Reaktionen, d.h. die Detektion biologisch relevanter Moleküle in definiertem Untersuchungsmaterial von herausragender Bedeutung. In diesem Rahmen wird die Entwicklung von sogenannten BioChips stetig vorangetrieben. Bei derartigen BioChips handelt es sich üblicherweise um miniaturisierte hybride Funktionselemente mit biologischen und technischen Komponenten, insbesondere auf einer Oberfläche (Außenoberfläche und/oder Innenoberfläche) eines BioChip-Grundmoduls immobilisierten Biomolekülen, die als spezifische Interaktionspartner dienen. Häufig weist die Struktur dieser Funktionselemente Reihen und Spalten auf. Man spricht dann von sogenannten "Chip-Arrays". Da tausende von biologischen bzw. biochemischen Funktionselementen auf einem Chip angeordnet sein können, werden diese in der Regel mit mikrotechnischen Methoden angefertigt.

Als biologische und biochemische Funktionselemente kommen insbesondere DNA, RNA, PNA, (bei Nukleinsäuren und ihren chemischen Derivaten können z.B. Einzelstränge, Triplex-Strukturen oder Kombinationen hiervon vorliegen), Saccharide, Peptide, Proteine (z.B. Antikörper, Antigene, Rezeptoren), Derivate der kombinatorischen Chemie (z.B. organische Moleküle), Zellbestandteile (z.B. Organellen), einzelne Zellen, mehrzellige Organismen sowie Zellverbände in Frage.

Die am weitesten verbreitete Variante von Biochips sind die sogenannten Microarrays. Dies sind kleine Plättchen ("Chips"), meist aus Glas, Gold, Kunstoff oder Silicium. Zum Nachweis entsprechender biologischer oder biochemischer Reaktionen werden beispielsweise kleine Mengen an unterschiedlichen Fängermolekülen, z.B. eine bekannte Nukleinsäuresequenz der Nucleosidbasen Adenin, Thymin, Guanin und Cytosin, in Form von kleinsten Tröpfchen punktförmig und matrizenartig, sogenannte Dots, auf der Oberfläche des BioChip-Grundmoduls als Sonden bzw. Fängermoleküle fixiert.

In der Praxis werden einige hundert bis einige tausend Tröpfchen pro Chip verwendet. Anschließend wird ein zu untersuchender Analyt, der üblicherweise fluoreszierend markiert werden kann, über diese Oberfläche gepumpt. Dabei kommt es im allgemeinen zu unterschiedlichen chemischen Reaktionen zwischen dem Analyten und den Tröpfchen, d.h. den fixierten Sonden bzw. Fängermolekülen. Wie bereits angeführt, wird zur Beobachtung dieser Reaktion der Analyt mit Farbstoffmolekülbausteinen, üblicherweise Fluorochromen markiert. Das Vorhandensein und die Intensität von Licht, das von den Fluorochromen emittiert wird, gibt Aufschluß über den Verlauf der Reaktion in den einzelnen Tröpfchen auf dem Substrat, so daß Rückschlüsse auf die Beschaffenheit des Analyten gezogen werden können. Wenn sich die entsprechenden Moleküle des fluoreszierend markierten Analyten mit den an der Oberfläche des Trägersubstrats immobilisierten Fängermolekülen umsetzen, kann durch optische Anregung mit einem Laser und Messung des entsprechenden Fluoreszenzsignals diese Reaktion nachgewiesen werden.

Eines der Hauptprobleme bei diesem Verfahren ist die Immobilisierung der Tröpfchen auf der Oberfläche des Biochips. Entscheidend sind hier zum einen eine ausreichende Dichte, mit der die Moleküle innerhalb der einzelnen Tröpfchen auf der Oberfläche des Substrats angeordnet werden können, und zum anderen eine ausreichende Stabilität der Immobilisierung, damit gewährleistet ist, daß sich die Moleküle innerhalb der Tröpfchen während der chemischen Reaktion nicht vom Substrat lösen. Sowohl die Dichte als auch die Stabilität der immobilisierten Moleküle hängen entscheidend von der Qualität der Substratoberfläche ab. Diese Oberflächenqualität wird heute im technischen Maßstab nicht optimal beherrscht, so daß sie einen begrenzenden Faktor für die Aussagefähigkeit der chemischen Experimente mit den derzeit verfügbaren Biochips darstellt.

Die Fängermoleküle bzw. Sonden, wie insbesondere Nukleinsäuren, werden üblicherweise nicht direkt auf die Glasoberfläche aufgetragen, vielmehr wird das eingesetzte Substrat zuvor beschichtet. Dafür verwendet man häufig Poly-L-Lysin, das Moleküle über ionische Wechselwirkungen an die Oberfläche bindet. Derart beschichtete Arrays können jedoch nur einmal verwendet weden, da beim Entfernen entsprechend hybridisierter Proben die immobilisierten Nukleinsäuren auf dem Chip ebenfalls abgelöst weden. Dieses Problem kann durch die Verwendung von Linkermolekülen wie z.B. Oligoethylenglykolderivaten umgangen werden. Des weiteren werden silylierte Glasträger eingesetzt, um modifizierte Nukleinsäuren kovalent an die Substratoberfläche zu binden und damit eine Wiederverwendung der Arrays zu ermöglichen.

In großem Maße werden derzeit Glasplättchen als Substrat eingesetzt, wie sie als Objektträger in der Mikroskopie verwendet werden. Diese Glasplättchen werden mit einer Oberflächenbeschichtung versehen, auf der die Moleküle der Tröpfchen möglichst gut haften sollen. Beispielsweise wird die Oberfläche der Glasplättchen mit einer monomolekularen Silan-Schicht versehen. Derartige Beschichtungen sind jedoch stets von der Qualität der Oberflächenbeschaffenheit des Substrats abhängig. Insofern bei einem Einsatz von Glas als Substrat derartige Glasoberflächen üblicherweise nicht ideal sind, wird mit Glassubstraten derzeit keine ausreichende Qualität sichergestellt.

Die zur Beschichtung eingesetzten Silane weisen an ihrem einen Ende eine funktionale Gruppe, die befähigt ist, mit den freien Hydroxylgruppen an der Glasoberfläche zu reagieren, und an ihrem anderen Ende eine funktionale Gruppe auf, die befähigt ist, als Verankerung für die Moleküle des zu immobilisierenden Fängermoleküls zu fungieren. Eine besondere Eigenschaft der Silane ist es, daß benachbarte Silanmoleküle mit Hilfe weiterer Gruppen untereinander polymerisieren, so daß auf der Glasoberfläche eine dichte, gleichmäßige monomolekulare Schicht entsteht, an der die in den Tröpfchen vorliegenden Fängermoleküle andocken können.

Diese Methode, bei welcher der die Substratoberfläche und die Fängermoleküle verbindende, dünne Film direkt auf das Substrat aufgetragen wird, hat jedoch zwei entscheidende Nachteile. Zum einen ist die Güte des Films und damit die Qualität des gesamten Biochips in hohem Maße von der Oberflächenqualität des Substrats abhängig. Diese ist bei dem heute vielfach verwendeten Glas uneinheitlich und oftmals mangelhaft, so daß es bei der Verwendung von Biochips häufig zu unbefriedigenden und schlecht reproduzierbaren Ergebnissen kommt. Zweitens kann bei der optischen Auswertung der Experimente mit den, an den Analyten gekoppelten Fluorochromen bzw. Farbstoffen (Marker) nicht die volle Lichtemission genutzt werden, da die Substratoberfläche nicht auf die optischen Eigenschaften der Farbstoffe abgestimmt ist und somit ein Teil des emittierten Lichtes verlorengeht.

Der vorliegenden Erfindung liegt somit die Aufgabe zugrunde, eine Vorrichtung bzw. "BioChip-Grundmodul" zum Nachweis biochemischer Reaktionen bereitzustellen, die bzw. das sich zum einen durch eine ausgezeichnete Oberflächenqualität des Substrats auszeichnen soll, um in entsprechenden biologisch-chemischen (molekularbiologischen) Analyse- bzw. Diagnoseverfahren gut reproduzierbare Ergebnisse zu liefern, und zum anderen in Abstimmung mit den optischen Eigenschaften der als Marker üblicherweise eingesetzten Fluorochrome eine optimale Lichtemission gewährleisten soll.

Diese Aufgabe wird durch die in den Ansprüchen gekennzeichneten Ausführungsformen gelöst.

Insbesondere wird eine Halbleitervorrichtung mit mehrschichtigem Aufbau bereitgestellt, welche mindestens ein Halbleitersubstrat und als äußere Schicht des Mehrschichtaufbaus eine SiO₂-Schicht in einer Dicke von größer als 5 nm umfaßt, wobei auf der SiO₂-Schicht eine Linkermolekül-Schicht auf der Basis einer Silicium-organischen Verbindung angeordnet ist und wobei die Silicium-organische Verbindung kovalent an die SiO₂-Schicht gebunden ist und weiter eine funktionelle Gruppe aufweist, die zur kovalenten Bindung mit als Sonden in biologisch-chemischen Reaktionen einsetzbaren Fängermolekülen befähigt ist.

Die erfindungsgemäße Halbleitervorrichtung eignet sich als "BioChip-Grundmodul" in Verfahren zum Nachweis biochemischer Reaktionen sowie hierfür insbesondere zur Untersuchung zur DNA-Hybridisierung, Protein-Protein-Wechselwirkungen und anderer Bindungsreaktionen im Bereich der Genom-, Proteomoder Wirkstoffforschung in Biologie und Medizin.

Die Verwendung eines Halbleitermaterials, vorzugsweise Silicium, Germanium oder Galliumarsenid, mehr bevorzugt Silicium, als Substrat und die Anordnung einer äußeren SiO₂-Schicht in einer Dicke von größer als 5 nm, vorzugsweise in einer Dicke im Bereich von 5 nm bis 1000 nm, besonders bevorzugt 5 nm bis 30 nm, d.h. einer nicht-nativen SiO₂-Schicht, in dem Mehrschichtaufbau ermöglicht eine Silanisierung, d.h. das Aufbringen einer Linkermolekül-Schicht auf der Basis einer Silicium-organischen Verbindung, welche weiter eine funktionelle Gruppe aufweist, die zur kovalenten Bindung mit als Sonden in biologisch-chemischen Reaktionen einsetzbaren Fängermolekülen befähigt ist, auf die SiO₂-Schicht in hoher Güte und Reproduzierbarkeit. Dies wiederum gewährleistet eine Immobilisierung entsprechender Fängermoleküle bzw. Sonden in hoher Stabilität und Dichte. Die optische Auswertung entsprechender Reaktionen auf dem fertigen Biochip kann dadurch besonders effektiv gestaltet werden. Die Möglichkeit, die physikalischen und optischen Eigenschaften der Oberfläche wesentlich zu verbessern, beruhen insbesondere auf der kristallinen Struktur des Halbleitermaterials und der äußeren, nicht-nativen SiO₂-Schicht.

Das Halbleitersubstrat kann beispielsweise eine Dicke im Bereich von 400 µm bis 700 µm, vorzugsweise 650 µm, aufweisen. Im Rahmen der vorliegenden Erfindung ist es jedoch nicht unbedingt notwendig, daß das gesamte Substrat aus einem Halbleitermaterial gebildet ist. Unter Umständen kann es auch ausreichend sein, beispielsweise ein Glassubstrat mit einer entsprechenden Halbleiterschicht wie z.B. einer Siliciumschicht zu versehen.

In einer bevorzugten Ausführungsform der erfindungsgemäßen Halbleitervorrichtung sind zwischen dem Halbleitersubstrat und der äußeren SiO₂-Schicht weiter eine oder mehrere Schichten i mit Brechnungsindizes nᵢ und Schichtdicken dᵢ dergestalt angeordnet, daß der Mehrschichtaufbau aus den einzelnen Schichten i in einer Abfolge alternierender Brechungsindizes nᵢ aufgebaut ist, so daß der Mehrschichtaufbau als optische Reflexschichtanordnung bei einer vorbestimmten Wellenlänge λ_{Detektion} wirkt. Vorzugsweise ist dabei der Mehrschichtaufbau in einer Abfolge von Schichten mit alternierenden Brechungsindizes nᵢ aufgebaut, wobei der Brechungsindex n₁ der äußeren SiO₂-Schicht kleiner als der Brechungsindex n₂ der in Normalenrichtung des Halbleitersubstrats angrenzenden Schicht ist. Der Mehrschichtaufbau aus den Schichten mit alternierenden Brechungsindizes kann auch derart ausgelegt sein, daß die Schichten einen sogenannten Bragg-Reflektor bilden, wobei die Schichten jeweils eine Schichtdicke von λ_{Detektion}/4 aufweisen. Derartige Bragg-Reflektoren finden beispielsweise bei vertikal emittierenden Halbleiterlaserdioden (vertical cavity suface emitting lasers, VCSELs) Verwendung und ermöglichen wellenlängenselektive Reflektoreinrichtungen mit hohen Reflexionskoeffizienten.

Derartige Anordnungen ermöglichen in besonders günstiger Weise eine optimale Ausnutzung der Lichtemission eines als Marker eingesetzten Fluorochroms, mit welchem ein entsprechender Analyt üblicherweise markiert ist. Unabhängig von den einzusetzenden Fluorochromen unterliegt der Mehrschichtaufbau keinen weiteren spezifischen Beschränkungen, solange der Mehrschichtaufbau in einer Abfolge von Schichten mit alternierenden Brechungsindizes vorliegt. Außerdem erhöht eine derartige Anordnung die Anzahl angeregter Fluorochrome, da das anregende Licht auch reflektiert wird die Schichtabfolge ein zweites mal durchdringt.

Als Fluorochrome können dabei die in derartigen Diagnoseverfahren, wie z.B. Hybridisierungsassays, üblichen Fluorochrome eingesetzt werden, wie z.B. Hydroxycumarin, Aminocumarin, Methoxycumarin, Cascade blue, Lucifer yellow, NBD, R-Phycoerythrin (PE), PE-Cy5 Konjugate, PE-Cy7 Konjugate, Red 613, Fluorescein, BODIPY-FL, Cy3, TRITC, X-Rhodamin, Lissamin Rhodamin B, PERCP, Texas Red, Cy5, Cy7, Allophycocyanin (APC), TruRed, APC-Cy7 Konjugate, Hoechst 33342, DAPI, Hoechst 33258, SYTOX Blue, Chromomycin A3, Mithramycin, YOYO-1, SYTOX Green, SYTOX Orange, Ethidiumbromid, 7-AAD, Acridin Orange, TOTO-1, TO-PRO-1, Thiazol Orange, Propidiumiodid (PI), TOTO-3, TOPRO-3, LDS 751, Indo-1, Fluo-3, DCFH, DHR, SNARF, Y66F, Y66H, EBFP, Wilde Type, GFPuv, ECFP, Y66W, S65A, S65C, S65L, S65T, EGFP, EYFP, DsRed, Monochlobiman und Calcein.

Die eine oder mehrere, zwischen dem Halbleitersubstrat und der äußeren SiO₂-Schicht angeordneten Schichten können beispielsweise aus der Gruppe, bestehend aus polykristallinem Silicium, Aluminium, Siliciumnitrid, Titannitrid, Tantalnitrid, Aluminiumoxid, Zinnoxid, Zinkoxid, Glas, Siliciumdioxid, Silicium-Germanium und Germanium, ausgewählt sein.

In einer besonders bevorzugten Ausführungsform umfaßt der Mehrschichtaufbau eine auf dem Halbleitersubstrat angeordnete erste Schicht, ausgewählt aus Siliciumnitrid, Titannitrid, Tantalnitrid, Aluminiumoxid, Zinnoxid, Zinkoxid, Glas, Siliciumdioxid, Silicium-Germanium und Germanium, und darauf angeordnet eine zweite Schicht aus polykristallinem Silicium. Ein derartiger Aufbau ermöglicht eine besonders hohe Selektivität bei der Anpassung an die als Marker üblicherweise verwendeten Fluorochrome. Die vorgenannten Schichten ergeben besonders glatte und homogene Oberflächen und weisen optische Eigenschaften auf, die sich durch die Gestaltung des Mehrschichtaufbaus, insbesondere deren entsprechenden Schichtdicken, an die später verwendeten Fluorochrome zur Auswertung der Analyseexperimente mittels des fertigen BioChips günstig anpassen lassen. Dabei kann die auf dem Halbleitersubstrat angeordnete erste Schicht eine Dicke im Bereich von 100 nm bis 200 nm und die darauf angeordnete zweite Schicht aus polykristallinem Silicium eine Dicke im Bereich von 10 nm bis 100 nm aufweisen. Beispielweise eigenen sich Anordnungen
"Bulk"-Si/ SiO₂ (188 nm)/ PolySi (30 nm)/ SiO₂ (10 nm)/Linkermolekülschicht bzw.
"Bulk"-Si/ SiO₂ (124 nm)/ PolySi (31 nm)/ SiO₂ (10 nm)/Linkermolekülschicht
für Cy3, Cy5, Texas Red bzw. FITC als Fluorochrome.

Die äußere SiO₂-Schicht ist vorzugsweise eine thermische SiO₂-Schicht oder eine CVD-SiO₂-Schicht. Besonders bevorzugt ist die äußere SiO₂-Schicht eine Schicht, die'in einer Sauerstoffumgebung thermisch, z.B. in einer RTP (Rapid Thermal Processing)-Anlage, hergestellt worden ist. Dabei lassen sich besonders hohe Temperaturen erzielen, bei denen die Oxidoberfläche verfließt und dadurch besonders glatt und homogen wird. Zu diesem Zweck können dotierte Gläser, wie z.B. BPSG (Bor-Phosphor-Silikat-Glas), BSG (Bor-Silikat-Glas oder PSG (Phosphor-Silikat-Glas). Nachdem diese Schicht ihre spezifizierte Dicke erreicht hat, kann das direkt anschließende Abkühlen des Mehrschichtaufbaus beispielsweise in einer Stickstoffumgebung erfolgen, welche die Oberfläche vor der Kontamination mit organischen und anorganischen Partikeln oder der ungewollten Reaktion mit in der Luft befindlichen Stoffen verhindert.

Unmittelbar an diesen Abkühlungsprozeß kann dann die weitere Beschichtung, d.h. das Aufbringen der Linkermolekül-Schicht, erfolgen. In diesem Falle kann eine für die optische Auswertung entsprechender Analyseverfahren äußerst vorteilhafte Oberfläche erhalten werden, da die Oxiddicke an die Wellenlänge der Fluorochrome, welche in den mit dem fertigen BioChip durchzuführenden Analyse- bzw. Diagnoseverfahren verwendet werden, angepaßt werden kann, so daß es zu einer maximalen Reflexion des emittierten Lichtes durch die Substratoberfläche kommt. Zusätzlich ermöglicht die glatte Oxidoberfläche eine sehr gleichmäßige Silan- bzw. Linkermolekülschicht, was für die Reproduzierbarkeit der Reaktionen mit dem Analyten und die anschließende Auswertung sehr günstig ist.

Die molekularen Verbindungen auf der Basis einer Silicium-organischen Verbindung, welche die Linkermolekül-Schicht aufbauen, unterliegen keiner spezifischen Beschränkung, solange sie befähigt sind, an die auf der Oberfläche der äußeren SiO₂-Schicht vorliegenden OH-Gruppen kovalent zu binden und weiter eine funktionelle Gruppe aufweisen, die zur kovalenten Bindung mit als Sonden in biologisch-chemischen Reaktionen einsetzbaren Fängermolekülen befähigt ist.

Derartige bifunktionelle Silicium-organische Verbindungen können beispielsweise Alkoxysilan-Verbindungen mit einer oder mehreren terminalen funktionalen Gruppen, ausgewählt aus Epoxy, Glycidyl, Chlor, Mercapto oder Amino, sein. Vorzugsweise ist die Alkoxysilan-Verbindung ein Glycidoxyalkylalkoxysilan, wie z.B. 3-Glycidoxypropyltrimethoxysilan, ein Mercaptoalkylalkoxysilan, wie z.B. γ-Mercaptopropyltrimethoxysilan, oder ein Aminoalkylalkoxysilan, wie z.B. N-β-(aminoethyl) γ-aminopropyltrimethoxysilan. Die Länge der als Spacer zwischen der funktionellen Gruppe, wie z.B. Epoxy bzw. Glycidoxy, welche mit dem Fängermolekül bzw. der Sonde bindet, und der Trialkoxysilangruppe wirkenden Alkylenreste unterliegt dabei keiner Beschränkung. Derartige Spacer können auch Polyethylenglykolreste sein.

In einer weiteren Ausführungsform der vorliegenden Erfindung weist das Halbleitersubstrat im Bereich des Mehrschichtaufbaus eine zumindest bereichsweise strukturierte Oberfläche auf. Vorzugsweise weist die strukturierte Oberfläche des Halbleitersubstrats eine Vielzahl periodisch oder stochastisch angeordneter Vertiefungen auf. Durch die Nano- bzw. Mikrostrukturierung des Halbleitersubstrats, welches als Trägereinrichtung für den darüber angeordneten Mehrschichtaufbau dient, kann das Reflexionsverhalten der bevorzugten erfindungsgemäßen Halbleitervorrichtung weiter verbessert werden. Insbesondere kann durch die Strukturierung des Halbleitersubstrats eine Vorzugsachse für die Reflexion vorgegeben werden. Die Reflexionseigenschaften der Halbleitervorrichtung können durch die Strukturierung derart gesteuert werden, daß ein möglichst großer Signalanteil der zu detektierenden Lichtintensität von dem Mehrschichtaufbau in einen Raumwinkel reflektiert wird, in welchem ein Lichtdetektor eines verwendeten Meßinstruments betriebsmäßig angeordnet ist.

Eine derartige Strukturierung des Halbleitersubstrats kann durch eine Plasmaätzung des Halbleitersubstrats, beispielsweise mit dem Advanced Silicon Etching Prozeß ("Bosch-Prozeß"), erzeugt werden. Dabei können insbesondere halbkugel-, paraboloid- und/oder pyramidenartigen Vertiefungen mit einer Tiefe in Normalenrichtung des Halbleitersubstrats von 1 µm bis 300 µm, vorzugsweise 10 µm bis 100 µm, erzeugt werden. Der Durchmesser der Vertiefungen senkrecht zu der Normalenrichtung des Halbleitersubstrats, d.h. in Waferebene, liegt üblicherweise in einem Bereich von 1 µm bis 300 µm, vorzugsweise 10 µm bis 100 µm. Um Vertiefungen in der zu strukturierenden Substratoberfläche zu erzeugen, kann z.B. auch eine KOH-Ätzung verwendet werden.

Bei einem solchen naßchemischen, anisotrop wirkenden Ätzprozeß auf beispielsweise einer Silizium (100)-Oberfläche entstehen invertierte Pyramiden mit einem Winkel von etwa 70°. Durch die geometrische Struktur kann die Reflektivität der Oberfläche z.B. orthogonal zu dieser weiter erhöht werden.

Besondere Vorteile ergeben sich, wenn ein Teil der fluoreszierenden Moleküle bzw. Fluorochrome in den Brennpunkt derartiger halbkugel-, paraboloid- und/oder pyramidenartigen Vertiefungen gebracht wird. Weist das Halbleitersubstrat beispielsweise eine paraboloidartige Vertiefung auf und befinden sich die fluoreszierenden Moleküle bzw. Fluorochrome in dem Brennpunkt (Fokus) des Paraboloids, so wird durch die Lichtreflexion an dem paraboloidartigen Mehrschichtaufbau ein näherungsweise planparalleles Strahlenbündel erzeugt, welches parallel zu der Normalenrichtung des Halbleitersubstrats gerichtet ist. Vorzugsweise wird somit die gesamte reflektierte Lichtintensität durch Konzentration in einen engen Raumwinkel einer Detektion zugänglich.

Im Anschluß an den Ätzprozeß werden die weiteren Schichten abgeschieden, so daß die Oberfläche des strukturierten Materials und insbesondere die Innenflächen der geometrischen Struktur damit beschichtet werden.

Wie bereits oben ausgeführt, werden die Fluorochrome bei Stattfinden einer Reaktion des Analytens, an den sie gebunden sind, mit den an die Linkerschicht-Moleküle gebundenen Sonden bzw. Fängermoleküle auf der Chipoberfläche immobilisiert. Eine derartige Ausführungsform, in der das Halbleitersubstrat im Bereich des Mehrschichtaufbaus eine solche, zumindest bereichsweise strukturierte Oberfläche aufweist, kann derart ausgelegt sein, daß sich ein Teil der Fluorochrome in der Nähe des Brennpunktes der geometrischen Struktur (Paraboloid, Halbkugel bzw. Pyramide) befindet. Die Emission der Fluorochrome erfolgt überwiegend in das optisch dichtere Medium. Findet das Auslesen der Anordnung im trockenen Zustand, z.B. in Umgebungsatmosphäre statt, so wird Licht vor allem in die äußere SiO₂-Schicht emittiert, an den weiteren Schichten des Mehrschichtaufbaus, beispielsweise in dieser Ausführungsform eine Aluminiumschicht, reflektiert und auf Grund der Paraboloidstruktur weitgehend als paralleles Lichtbündel orthogonal zur Oberfläche abgestrahlt. Dadurch kann eine weitere Verbesserung im Vergleich zu planaren Glas-Chips bewirkt werden, bei denen ein erheblicher Anteil des Lichtes in den Glas-Chip emittiert wird und dadurch nicht mehr mit der Detektionseinheit über der Chip-Oberfläche erfaßt werden kann.

Für Analyseverfahren werden üblicherweise kleine Mengen unterschiedlicher einzelsträngiger Nukleinsäuremoleküle als Fängermoleküle bzw. Sonden über kovalente Bindung an die Linkermoleküle auf die Chip-Oberfläche aufgebracht. Sie werden dabei punktförmig in einem geordneten Raster (Array) von z.B. einigen 10, 100 oder 1000 Punkten (Dots) auf der Oberfläche immobilisiert. Die typische Größe (= Durchmesser) solcher Dots beträgt etwa 10 bis 300 µm. Bei sehr kleinen Durchmessern der halbkugel-, paraboloid- und/oder pyramidenartigen Vertiefungen befinden sich einige hundert bis tausend als Reflektor dienende Vertiefungen unter einem Messpunkt (Dot), der statistisch über diese verteilt ist. Wenn der Durchmesser der vorgenannten Strukturen im Bereich der Dot-Größe liegt, sind nur wenige solcher Vertiefungen unter der Fläche eines Dots angeordnet. Typischerweise befindet sich dann ein Dot auf einer Reflexionsstruktur. Das Aufbringen der Fängermoleküle kann dann in vorteilhafter Weise justiert zu den Strukturen auf dem Chip durchgeführt werden.

Gegenstand der vorliegenden Erfindung ist weiter eine wie vorstehend beschriebene Halbleitervorrichtung ("BioChip-Grundmodul"), bei der an die Linkermoleküle kovalent Fängermoleküle gebunden sind, ausgewählt aus der Gruppe, bestehend aus DNA, Proteinen und Liganden. Vorzugsweise sind die Fängermoleküle Oligonukleotidsonden, die über endständige Amino- oder Thiolgruppen an die Linkermoleküle gebunden sind.

Ein weiterer Gegenstand der vorliegenden Erfindung betrifft ein Verfahren zur Herstellung einer wie vorstehend beschriebenen Halbleitervorrichtung mit mehrschichtigem Aufbau, umfassend die Schritte:
- Bereitstellen eines Halbleitersubstrats,
- Anordnen einer SiO₂-Schicht in einer Dicke von größer als 5 nm als äußere Schicht des Mehrschichtaufbaus, und
- Aufbringen einer Linkermolekül-Schicht auf der Basis einer Silicium-organischen Verbindung auf der äußeren SiO₂-Schicht durch kovalentes Binden einer bifunktionellen Silicium-organischen Verbindung an die SiO₂-Schicht, wobei die Silicium-organische Verbindung nach kovalenter Bindung an die SiO₂-Schicht eine funktionelle Gruppe aufweist, die zur kovalenten Bindung mit als Sonden in biologisch-chemischen Reaktionen einsetzbaren Fängermolekülen befähigt ist.

In einer bevorzugten Ausführungsform des erfindungsgemäßen Verfahrens werden zwischen dem Halbleitersubstrat und der äußeren SiO₂-Schicht eine oder mehrere Schichten i mit Brechnungsindizes nᵢ und Schichtdicken dᵢ dergestalt angeordnet, daß der Mehrschichtaufbau aus den einzelnen Schichten i in einer Abfolge alternierender Brechungsindizes nᵢ aufgebaut ist, so daß der Mehrschichtaufbau als optische Reflexschichtanordnung bei einer vorbestimmten Wellenlänge λ_{Detektion} wirkt.

Ein besonderer Vorteil besteht dabei in der Möglichkeit einer engen, definierten zeitlichen Kopplung zwischen dem Aufbringen der äußeren SiO₂-Schicht auf das Halbleitersubstrats, dem gegebenenfalls ein Aufbringen von einer oder mehreren Schichten aus der Gruppe, bestehend aus polykristallinem Silicium, Aluminium, Siliciumnitrid, Titanoxid, Tantaloxid, Titannitrid, Tantalnitrid, Aluminiumoxid, Zinnoxid, Zinkoxid, Glas, Siliciumdioxid, Silicium-Germanium und Germanium, auf das Halbleitersubstrat vorausgeht, und dem Aufbringen der Linkermolekülschicht, d.h. der Silanisierung. Durch diese enge Kopplung kann vermieden werden, daß die äußere SiO₂-Oberfläche nach ihrer Fertigstellung längere Zeit in Umgebungsluft gehandhabt oder gelagert und so
dem Risiko einer Kontaminierung mit Partikeln ausgesetzt wird, die eine anschließende Beschichtung hoher Güte
und Reproduzierbarkeit unmöglich macht.

Vorzugsweise wird insbesondere der Schritt des Anordnens der äußeren SiO₂-Schicht und des kovalenten Anbindens der Linkermolekül-Schicht in demselben Reaktor in der Gasphase durchgeführt. Dadurch wird erreicht, daß der gesamte Herstellungsprozeß durchgehend kontrollierten Bedingungen unterliegt, was sich äußerst vorteilhaft auf die Qualität und Reproduzierbarkeit auswirkt.

In einer anderen Ausführungsform kann der Schritt des kovalenten Anbindens der Linkermolekül-Schicht auch in flüssiger Phase durchgeführt werden.

Das erfindungsgemäße Verfahren kann ferner den Schritt des kovalenten Bindens von Fängermolekülen, ausgewählt aus DNA, Proteinen und Liganden, vorzugsweise endständige Amino- oder Thiolgruppen aufweisende Oligonukleotide, an die Linkermoleküle zur Herstellung eines einsatzfähigen, fertigen BioChips umfassen.

Vorzugsweise wird in dem Verfahren Silicium als Halbleitersubstrat verwendet. Reinstsilicium wird insbesondere in Form von Wafern erzeugt, die z.B. 6 oder 8 Zoll Durchmesser aufweisen. Diese Wafer können in einfacher Weise zu einzelnen Chips vereinzelt werden. Die Herstellung der erfindungsgemäßen Halbleitervorrichtung läßt eine Vereinzelung sowohl vor als auch nach der Herstellung der erfindungsgemäßen Halbleitervorrichtung ("BioChip-Grundmodul") zu.

Ein Aspekt der Erfindung wird nachfolgend unter Bezugnahme auf die begleitende Zeichnung beispielhaft beschrieben. Es zeigt:

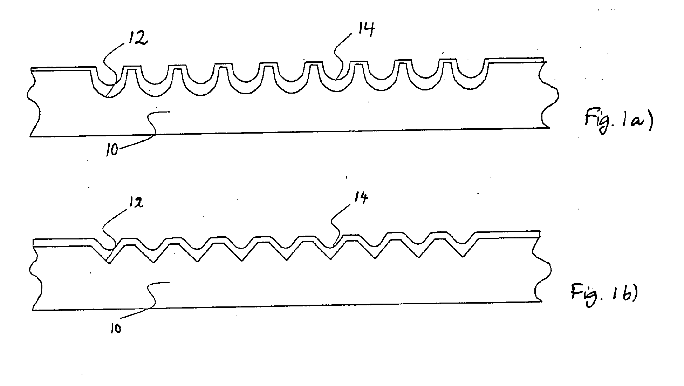

- Fig. 1a: eine schematische Schnittansicht einer bevorzugten Ausführungsform einer erfindungsgemäßen Halbleitervorrichtung mit paraboloid-strukturiertem Halbleitersubstrat; und
- Fig. 1b: eine schematische Schnittansicht einer weiteren bevorzugten Ausführungsform einer erfindungsgemäßen Halbleitervorrichtung mit pyramiden-strukturiertem Halbleitersubstrat.

In Fig. 1a ist eine Ausführungsform einer erfindungsgemäßen Halbleitervorrichtung stark schematisiert im Schnitt dargestellt. Als Trägermaterial dient ein in der Halbleiterindustrie übliches Siliciumsubstrat 10, welches als einkristalliner Halbleiterwafer geliefert wird. Die Prozeßoberfläche des Substrats 10 wurde einem Ätzschritt unterworfen, um eine Vielzahl regelmäßig angeordneter paraboloidartiger Vertiefungen 12 in dem Substrat 10 auszubilden. Die in Fig. 1a dargestellte vertikale, d.h. entlang der Normalenrichtung des Substrats 10 verlaufende Abmessung der Vertiefungen 12 ist zur besseren graphischen Darstellbarkeit verglichen mit der Substratdicke stark vergrößert abgebildet. Vorzugsweise wird eine Vielzahl der Vertiefungen 12 matrixartig in dem Substrat 10 angeordnet, um einen vorbestimmten Flächenbereich zu bedecken.

Durch geeignete Prozeßschritte wird auf die derart strukturierte Substratoberfläche des Substrats 10 ein Mehrschichtaufbau 14 angeordnet, dessen äußere Schicht eine zumindest 5 nm dicke SiO₂-Schicht mit daran angeordneter Linkermolekül-Schicht ist. Wie in Fig. 1 dargestellt folgt der Mehrschichtaufbau 14 dem vertikalen Verlauf der strukturierten Substratoberfläche. Vorzugsweise wird die Schichtdicke der äußeren SiO₂-Schicht des Mehrschichtaufbaus 14 derart dimensioniert, daß die Linkermolekül-Schicht durch den optischen Brennpunkt (Fokus) der paraboloidartigen Vertiefungen 12 verläuft. Alternativ kann auch eine optisch transparente Hilfsschicht (eine sogenannte Spacer-Schicht) insbesondere unmittelbar unterhalb der äußeren SiO₂-Schicht angeordnet werden, um die Linkermolekül-Schicht genau soweit von der Substratoberfläche zu beabstanden, daß diese durch den optischen Brennpunkt der Vertiefungen 12 verläuft. Die fluoreszierenden Moleküle bzw. Fluorochrome werden vorzugsweise in den Bereichen der optischen Brennpunkte der Vertiefungen 12 angeordnet.

Von den fluoreszierenden Molekülen bzw. Fluorochromen in Richtung Substrat 10 emittiertes Licht wird von dem Mehrschichtaufbau 14 als planparalleles Strahlenbündel in Richtung der Normalenrichtung des Substrats 10 reflektiert. Ein in dieser Richtung angeordneter Lichtdetektor einer optischen Meßvorrichtung kann aufgrund der so ausgebildeten Reflexionscharakteristik der Halbleitervorrichtung besonders effizient arbeiten. Im einfachsten Fall besteht ein bevorzugter Mehrschichtaufbau 14 der in Fig. 1 dargestellten Ausführungsformen aus einer dünnen reflektierenden Metallschicht, vorzugsweise einer Aluminiumschicht, und einer darauf angeordneten äußeren SiO₂-Schicht mit der Linkermolekül-Schicht.

In Fig. 1b ist eine weitere Ausführungsform einer erfindungsgemäßen Halbleitervorrichtung dargestellt. Diese Ausführungsform unterscheidet sich von der anhand von Fig. 1a beschriebenen Ausführungsform lediglich durch die geometrische Form der Vertiefungen 12, welche durch einen KOH-Ätzschritt auf einem (100)-orientierten Siliciumsubstrat pyramidenförmig ausgebildet sind.

## Patentansprüche

1. Halbleitervorrichtung mit mehrschichtigem Aufbau, mindestens umfassend ein Halbleitersubstrat und als äußere Schicht des Mehrschichtaufbaus eine SiO₂-Schicht in einer Dicke von größer als 5 nm, wobei auf der SiO₂-Schicht eine Linkermolekül-Schicht auf der Basis einer Silicium-organischen Verbindung angeordnet ist und wobei die Silicium-organische Verbindung kovalent an die SiO₂-Schicht gebunden ist und weiter eine funktionelle Gruppe aufweist, die zur kovalenten Bindung mit als Sonden in biologisch-chemischen Reaktionen einsetzbaren Fängermolekülen befähigt ist.

2. Halbleitervorrichtung nach Anspruch 1, wobei zwischen dem Halbleitersubstrat und der äußeren SiO₂-Schicht eine oder mehrere Schichten i mit Brechnungsindizes nᵢ und Schichtdicken dᵢ dergestalt angeordnet sind, daß der Mehrschichtaufbau aus den einzelnen Schichten i in einer Abfolge alternierender Brechungsindizes nᵢ aufgebaut ist, so daß der Mehrschichtaufbau als optische Reflexschichtanordnung bei einer vorbestimmten Wellenlänge λ_{Detektion} wirkt.

3. Halbleitervorrichtung nach Anspruch 2, wobei der Mehrschichtaufbau in einer Abfolge von Schichten mit alternierenden Brechungsindizes nᵢ aufgebaut ist, wobei der Brechungsindex n₁ der äußeren SiO₂-Schicht kleiner als der Brechungsindex n₂ der in Normalenrichtung des Halbleitersubstrats angrenzenden Schicht ist.

4. Halbleitervorrichtung nach Anspruch 2 oder 3, wobei die eine oder mehrere, zwischen dem Halbleitersubstrat und der äußeren SiO₂-Schicht angeordneten Schichten aus der Gruppe, bestehend aus polykristallinem Silicium, Aluminium, Titanoxid, Tantaloxid, Siliciumnitrid, Titannitrid, Tantalnitrid, Aluminiumoxid, Zinnoxid, Zinkoxid, Glas, Siliciumdioxid, Silicium-Germanium und Germanium ausgewählt sind.

5. Halbleitervorrichtung nach einem der Ansprüche 1 bis 4, umfassend eine auf dem Halbleitersubstrat angeordnete erste Schicht, ausgewählt aus Siliciumnitrid, Titannitrid, Titanoxid, Tantaloxid, Tantalnitrid, Aluminiumoxid, Zinnoxid, Zinkoxid, Glas, Siliciumdioxid, Silicium-Germanium und Germanium, und darauf angeordnet eine zweite Schicht aus polykristallinem Silicium.

6. Halbleitervorrichtung nach einem der Ansprüche 1 bis 5, wobei die äußere SiO₂-Schicht eine thermische SiO₂-Schicht oder eine CVD-SiO₂-Schicht ist.

7. Halbleitervorrichtung nach einem der Ansprüche 1 bis 6, wobei das Halbleitersubstrat Silicium ist.

8. Halbleitervorrichtung nach einem der Ansprüche 1 bis 7, wobei das Halbleitersubstrat eine Dicke im Bereich von 400 µm bis 700 µm, vorzugsweise 650 µm, aufweist.

9. Halbleitervorrichtung nach einem der Ansprüche 1 bis 8, wobei die äußere SiO₂-Schicht eine Dicke im Bereich von 5 nm bis 5000 nm, vorzugsweise 5 nm bis 30 nm aufweist.

10. Halbleitervorrichtung nach einem der Ansprüche 5 bis 9, wobei die auf dem Halbleitersubstrat angeordnete erste Schicht eine Dicke im Bereich von 100 nm bis 200 nm und die darauf angeordnete zweite Schicht aus polykristallinem Silicium eine Dicke im Bereich von 10 nm bis 100 nm aufweisen.

11. Halbleitervorrichtung nach einem der Ansprüche 1 bis 10, wobei die Linkermolekül-Schicht auf Basis einer Alkoxysilan-Verbindung mit einer oder mehreren terminalen funktionalen Gruppen, ausgewählt aus Epoxy, Glycidyl, Chlor, Mercapto oder Amino, ist.

12. Halbleitervorrichtung nach Anspruch 11, wobei die Alkoxysilan-Verbindung ein Glycidoxyalkylalkoxysilan, ein Mercaptoalkylalkoxysilan oder Aminoalkylalkoxysilan ist.

13. Halbleitervorrichtung nach einem der vorhergehenden Ansprüche, wobei das Halbleitersubstrat im Bereich des Mehrschichtaufbaus eine zumindest bereichsweise strukturierte Oberfläche aufweist.

14. Halbleitervorrichtung nach Anspruch 13, wobei die strukturierte Oberfläche des Halbleitersubstrats eine Vielzahl periodisch oder stochastisch angeordneter Vertiefungen aufweist.

15. Halbeitervorrichtung nach Anspruch 14, wobei die Vertiefungen halbkugel-, paraboloid- und/oder pyramidenartige Vertiefungen mit einer Tiefe in Normalenrichtung des Halbleitersubstrats von 1 µm bis 300 µm, vorzugsweise 10 µm bis 100 µm sind.

16. Halbleitervorrichtung nach Anspruch 14 oder 15, wobei der Durchmesser der Vertiefungen senkrecht zu der Normalenrichtung des Halbleitersubstrats in einem Bereich von 1 µm bis 300 µm, vorzugsweise 10 µm bis 100 µm liegt.

17. Halbleitervorrichtung nach einem der Ansprüche 1 bis 16, wobei an die Linkermoleküle kovalent Fängermoleküle gebunden sind, ausgewählt aus der Gruppe, bestehend aus DNA, Proteinen und Liganden.

18. Halbleitervorrichtung nach Anspruch 17, wobei die Fängermoleküle Oligonukleotidsonden sind, die über endständige Amino- oder Thiolgruppen an die Linkermoleküle gebunden sind.

19. Verfahren zur Herstellung der Halbleitervorrichtung mit mehrschichtigem Aufbau nach einem der vorhergehenden Ansprüche, umfassend die Schritte:
- Bereitstellen eines Halbleitersubstrats,
- Anordnen einer SiO₂-Schicht in einer Dicke von größer als 5 nm als äußere Schicht des Mehrschichtaufbaus, und
- Aufbringen einer Linkermolekül-Schicht auf der Basis einer Silicium-organischen Verbindung auf der äußeren SiO₂-Schicht durch kovalentes Binden einer bifunktionalen Silicium-organischen Verbindung an die SiO₂-Schicht, wobei die Silicium-organische Verbindung nach kovalenter Bindung an die SiO₂-Schicht eine funktionelle Gruppe aufweist, die zur kovalenten Bindung mit als Sonden in biologisch-chemischen Reaktionen einsetzbaren Fängermolekülen befähigt ist.

20. Verfahren nach Anspruch 19, worin zwischen dem Halbleitersubstrat und der äußeren SiO₂-Schicht eine oder mehrere Schichten i mit Brechnungsindizes nᵢ und Schichtdicken dᵢ dergestalt angeordnet werden, daß der Mehrschichtaufbau aus den einzelnen Schichten i in einer Abfolge alternierender Brechungsindizes nᵢ aufgebaut ist, so daß der Mehrschichtaufbau als optische Reflexschichtanordnung bei einer vorbestimmten Wellenlänge λ_{Detektion} wirkt.

21. Verfahren nach Anspruch 19 oder 20, worin die äußere SiO₂-Schicht thermisch oder mittels CVD-Verfahren, vorzugsweise thermisch, abgeschieden wird.

22. Verfahren nach einem der Ansprüche 19 bis 21, worin der Schritt des Anordnens der äußeren SiO₂-Schicht und des kovalenten Anbindens der Linkermolekül-Schicht in demselben Reaktor in der Gasphase durchgeführt wird.

23. Verfahren nach einem der Ansprüche 19 bis 21, worin der Schritt des kovalenten Anbindens der Linkermolekül-Schicht in flüssiger Phase durchgeführt wird.

24. Verfahren nach einem der Ansprüche 19 bis 23, ferner umfassend den Schritt des kovalenten Bindens von Fängermolekülen, ausgewählt aus DNA, Proteinen und Liganden, vorzugsweise endständige Amino- oder Thiolgruppen aufweisende Oligonukleotiden, an die Linkermoleküle.

25. Verfahren nach einem der Ansprüche 19 bis 24, worin das Halbleitersubstrat vor dem Aufbringen weiterer Schichten derart strukturiert wird, daß mindestens eine Substratoberfläche eine Vielzahl periodisch oder stochastisch angeordneter Vertiefungen aufweist.

26. Verfahren nach Anspruch 25, worin die Strukturierung mittels Plasmaätzung, vorzugweise mittels eines Advanced Silicon Etching-Prozesses, oder KOH-Ätzung erzeugt wird.

27. Verwendung der Halbleitervorrichtung nach einem der Ansprüche 1 bis 18 als BioChip-Grundmodul in Verfahren zum Nachweis biochemischer Reaktionen sowie hierfür insbesondere zur Untersuchung zur DNA-Hybridisierung, Protein-Protein-Wechselwirkungen und anderer Bindungsreaktionen im Bereich der Genom-, Proteom- oder Wirkstoffforschung in Biologie und Medizin.
